# EUROPEAN PATENT SPECIFICATION

(11) **EP 2 753 398 B1**
(45) Date of publication and mention of the grant of the patent: **08.01.2020**
(21) Application number: 12830817.8
(22) Date of filing: 10.09.2012
(51) Int. Cl.: A61N 1/18, A61N 1/05, A61B 5/00, A61N 1/36

(54) **SYSTEM FOR PROVIDING TARGETED ELECTRICAL STIMULATION TO TISSUE**
SYSTEM ZUR GEZIELTEN ELEKTRISCHEN STIMULATION EINES GEWEBES
SYSTÈME SERVANT À ENVOYER UNE STIMULATION ÉLECTRIQUE CIBLÉE VERS DES TISSUS

(30) Priority: 08.09.2011 US 201161532550 P
(43) Date of publication of application: 16.07.2014
(73) Proprietor: Checkpoint Surgical, LLC, Cleveland, OH 44122 (US)
(72) Inventor: STROTHER, Robert, B., Willoughby Hills, OH 44904 (US); SAKAI, Jonathan, Fairview Park, OH 44126 (US)
(74) Representative: Müller-Boré & Partner Patentanwälte PartG mbB
(86) International application number: PCT/US2012/054403
(87) International publication number: WO 2013/036908

(56) References cited:
- US-A- 4 892 105
- US-A- 5 012 816
- US-A- 6 159 194
- US-A1- 2006 200 219
- US-A1- 2006 200 219
- US-A1- 2011 125 051
- US-A1- 2011 125 051
- US-B2- 6 766 202

## Description

### Background of the Invention

Embodiments according to the present description relate to electrical stimulation devices, and more particularly to configurations of electrical stimulation probes and communication of information from an electrical stimulation device.

Prior electrical stimulation apparatuses are known. Generally, however, only a portion of the probe's conductive surface (the end of the tip in contact with the tissue and the small meniscus of body fluid that forms to that tip) delivers stimulus current to tissue as the device is used in open surgical procedures. In arthroscopic procedures, however, at least some of the probe, and perhaps the entire probe, may be exposed to fluid (nominally saline), leading to concerns consistent with informal intraoperative experience that the stimulus current may be shunted away from the 5mm long conductive tip by the surrounding saline. Such shunting reduces the current flowing from the tip into the tissue to a level too low to desirably activate target nerves innervating the tissue, even when the probe is contacting tissue directly over the target nerve.

US 2011/125051) describes a stimulation control device may incorporate a range of low and high intensity stimulation to provide a stimulation and evaluation of both nerves and muscles. A stimulation control device is removably coupled to a surgical device or is imbedded within the medical device to provide a stimulation and treatment medical device. A disposable hand held stimulation system includes an operative element extending from the housing, the housing includes a visual indication to provide feedback or status to the user.

US 6,159,194 describes methods for performing electrosurgical interventions, such as selectively contracting soft collagen tissue and other body structures, while limiting thermal damage or molecular dissociation of such tissue and limiting the thermal damage to tissue adjacent to and underlying the treatment site. A method for contracting collagen tissue at a target site on or within a patient's body comprises heating an electrically conducting fluid in the region of the target site; and directing the heated electrically conducting fluid onto tissue at the target site to induce contraction of collagen fibers in said tissue.

US 2006/200219 describes a system for differentiating or identifying targeted tissue regions locally innervated by a targeted nerve, the system comprising a hand held instrument including an electrode configuration for applying an electrical stimulation current to a tissue region, the hand held instrument including a handle, an electrical stimulation control circuitry carried within the handle, and at least one controller carried on the handle and coupled to the control circuitry for selectively altering at least one operating parameter of the control circuitry, and a second device that indicates a physiologic response to the presence or absence of the electrical stimulation current..

### Summary of the Invention

Embodiments of a probe according to the present description include a cathode located on a distal tip of a probe and a proximally located anode on the probe, which is spaced some insulative distance from the cathode. A preferred cathode has a conductive surface area of preferably less than or equal to ten square millimeters. A preferred anode provides a cylindrical electrically conductive surface that is greater in area than the exposed, electrically conductive surface of the cathode, preferably greater than nine times that of the cathode. The insulative distance between the exposed electrically conductive cathode surface and the exposed electrically conductive anode surface is preferably at least three millimeters and more preferably five to ten millimeters.

Probe constructions according to the present decription may be beneficially exploited in arthroscopic procedures, in which targeted electrical stimulation is desirable to be applied to tissue that may be covered in a pool or volume of conductive solution, such as saline solution. Preferably, in use, both electrode surfaces would be surrounded by saline which was previously or simultaneously infused to fill a surgical site. Alternatively, only a portion of the electrically conductive anode surface may be disposed within the saline solution. Preferably, all probe features are sized to enable passage through a 5mm (inner diameter) arthroscopic surgical cannula. Although both electrodes are physically near one another, the construction and usage are such that the probe generates an essentially monopolar stimulating field. Key construction & usage details include: the Anode (return) sleeve is spaced at least 3 mm away from the Cathode at the end of the probe, the sleeve is surrounded by saline, the sleeve has a much greater surface area than the Cathode, and the sleeve is usually not directly touching excitable tissue.

A system according to the present description may include an improved user interface disposed at or near a junction point of a probe section of an electrical stimulator and a handle section thereof.

### Brief Description of the Drawings

Figure 1A is a perspective view of an electrical stimulator incorporating embodiments according to the present description.
Figure 1B is a perspective close-up view of a probe construction according to the present description.
Figure 2A is a cross-section view of a first embodiment of a probe construction according to the present description taken along line 2A-2A of Figure 1B.
Figure 2B is a cross-section view of a second embodiment of a probe construction according to the present description.
Figure 2C is a cross-section view of a third embodiment of a probe construction according to the present description.
Figure 3 is a partial cutaway right elevation view of the embodiment of Figure 1A.
Figure 4 is a partial cutaway cross-section view taken along line 4-4 of Figure 1A.
Figure 5 is a partial cutaway cross-section view taken along line 5-5 of Figure 4.

### Description of the Preferred Embodiment

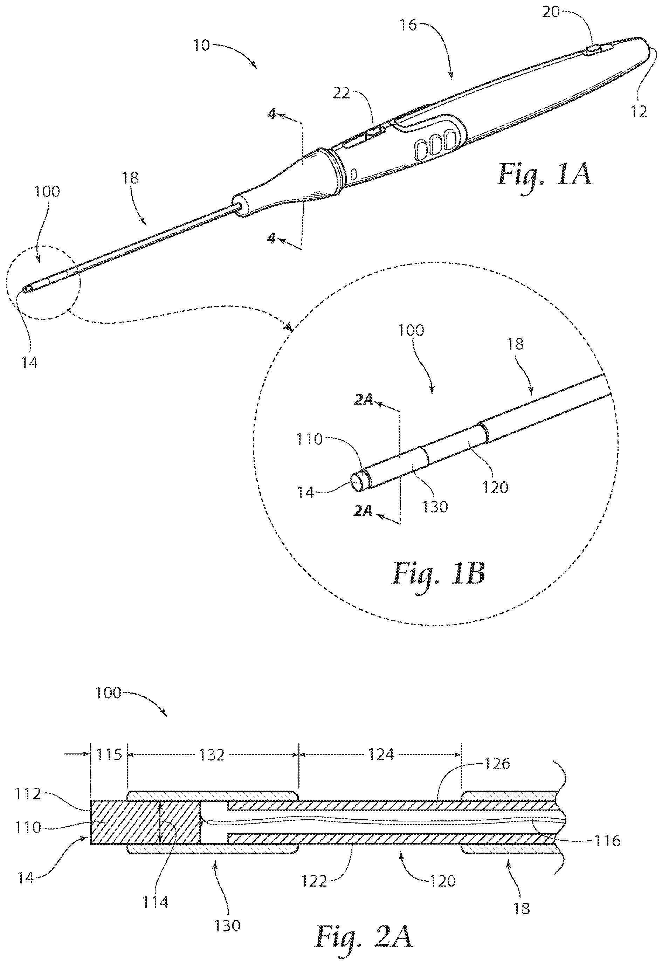

Turning now to the figures, Figure 1A depicts an electrical stimulator device 10 incorporating embodiments according to the present description. The stimulator 10 extends from a proximal end 12 to a distal end 14. A handle 16 extends from the proximal end 12 towards the distal end 14. The handle 16 preferably houses electrical stimulation generation circuitry, which may receive control inputs from user input controls, such as an amplitude adjustment switch 20 and/or a preferably continuous pulse width adjustment slide switch 22. The handle 16 may also include a low (relative to the rest of the handle 16) durometer grip portion 24 to aid in manual manipulation.

A probe 18 extends from the distal end 14 towards the proximal end 12 and is coupled to the handle 16. Provided at and extending from the distal end 14 towards the proximal end 12 is a preferred probe configuration 100, as seen in Figure 1B. With reference also to Figure 2A, a preferred probe configuration 100 according to the present description includes a first electrode 110, a second electrode 120, and a first insulative portion 130. The first electrode 110 has a first external electrically conductive surface 112, having a first exposed electrode surface area, which is disposed at the distal end 14 of the probe 18. The electrically conductive surface 112 is electrically coupled to the electrical stimulation generation circuitry that is housed in the handle 16. The first surface 112, which preferably serves as a stimulation cathode, has a preferred diameter 114, such as about two to about three millimeters, and more preferably about 2.5 mm. The second electrode 120 has a second external electrically conductive surface 122 having a second exposed electrode surface area. The second conductive surface 122, which is preferably at least substantially cylindrical, is spaced proximally from the first conductive surface 112 by the first insulative portion 130. The second conductive surface 122 may extend from the first insulative portion 130 for an electrode length 124. The first conductive surface 112 and the second conductive surface 122 may be formed of any desirable material, but preferably are stainless steel.

The first conductive surface 112 also preferably extends along a desirable length 115, longitudinally distally from the first insulative portion 130. A preferred range of lengths 115 is from 0.00 millimeters to about 6.00 millimeters, with about 0.50 to about 5.10 millimeters being most preferred. The length 115 may be selected on the basis of a desired expected charge density to be experienced during use. Preferred cathodic charge density may be in the range of about 0.5 µC/mm2 to about 2.0 µC/mm2, while preferred anodic charge density may be in the range of greater than zero µC/mm2 to about 0.6 µC/mm2. During use, it may be desirable to prevent tissue damage, in which case the size of the first electrode 110 and/or second electrode 120 should be sized accordingly. A preferred surface area of a cathodic surface, either the conductive surface 112 of the first electrode 110 or the conductive surface 122 of the second electrode 120, is generally about 1 mm² to about 20 mm², with about 1.6 mm² to about 16 mm² being most preferable.

Preferably, the second exposed surface area of the second conductive surface 122 is greater than the first exposed surface area of the first conductive surface 112. More preferably, for an expected depth of saline solution disposed on a stimulation side of the animal tissue to be stimulated, a greater amount of the second surface area is exposed to the solution than the whole of the first surface area. For example, if the first exposed surface area of the first conductive surface 112 is about seven square millimeters, then the electrode length 124 should be selected so as to establish a second exposed surface area of the second conductive surface 122 that is greater than seven square millimeters. Even more preferably, the second exposed surface area of the second conductive surface 122 is more than five times the first exposed surface area, and most preferably, it is at least nine times the first exposed surface area. Thus, in our example, if the first surface area is seven square millimeters, the second surface area is most preferably at least 63 square millimeters.

The second conductive surface 122 may be formed as an external surface of a conductive cylinder or other tubular member 126, which may extend a majority of a longitudinal length of the probe 18. The second conductive surface 122 is electrically coupled to the electrical stimulation generation circuitry contained within the handle 16, and the surface 122 preferably serves as a stimulation anode. If the member 126 is used, the first conductive surface 112 may be coupled to the stimulation circuitry by an insulated, electrically conductive filament or wire 116, which is soldered or otherwise electrically coupled to the first electrode 110.

The first insulative portion 130 is disposed proximal to the first conductive surface 112 and operates to prevent direct proximal conduction of electrical current between the first conductive surface 112 and the second conductive surface 122 if the probe 18 is disposed in air. The first insulative portion 130 provides a minimum insulative gap 132 between the first conductive surface 112 and the second conductive surface 122. The minimum insulative gap 132 is preferably three millimeters, but even more preferably five millimeters.

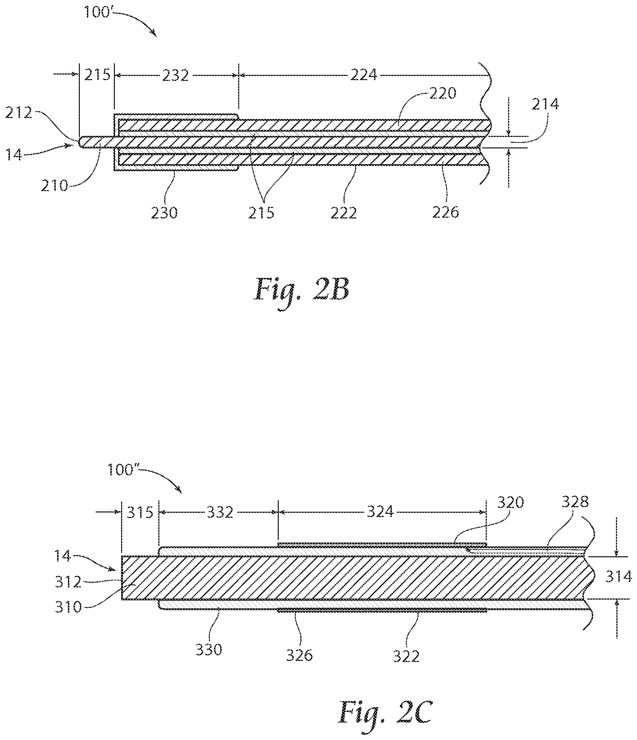

A second preferred probe configuration 100' is shown in Figure 2B, where like numbers refer to like structure from the first embodiment 100. This embodiment 100', like the first embodiment 100, has a first electrode 210, a second electrode 220, and a first insulative portion 230. The dimensions of the second embodiment 100' are preferably similar to or the same as the first embodiment 100, but the diameter 214 of the first conductive surface 212 may be less than 2.5 mm, such as about 1 mm.

The first conductive surface 212 also preferably extends along a desirable length 215, longitudinally distally from the first insulative portion 230. A preferred range of lengths 215 is from 0.00 millimeters to about 6.00 millimeters, with about 0.50 to about 5.10 millimeters being most preferred. The length 215 may be selected on the basis of a desired expected charge density to be experienced during use. Preferred cathodic charge density may be in the range of about 0.5 µC/mm2 to about 2.0 µC/mm2, while preferred anodic charge density may be in the range of greater than zero µC/mm2 to about 0.6 µC/mm2. During use, it may be desirable to prevent tissue damage, in which case the size of the first electrode 210 and/or second electrode 220 should be sized accordingly. A preferred surface area of a cathodic surface, either the conductive surface 212 of the first electrode 210 or the conductive surface 222 of the second electrode 220, is generally about 1 mm² to about 20 mm², with about 1.6 mm² to about 16 mm² being most preferable.

The method of construction of the second embodiment 100' differs slightly from the first embodiment 100, however. While the second electrode 220 may be formed generally as a conductive tubular member 226, the first electrode 210 may be extend throughout at least a majority of the tubular member 226, but electrically insulated from contact therewith by an insulative sheath or layer 215. At the distal end of the tubular member 226, the insulative portion 230 preferably extends over such distal end and along the minimum insulative gap distance 232.

A third preferred probe configuration 100" is shown in Figure 2C, where like numbers refer to like structure from the first embodiment 100. This embodiment 100", like the first embodiment 100, has a first electrode 310, a second electrode 320, and a first insulative portion 330. The dimensions of the second embodiment 100" are preferably similar to or the same as the first embodiment 100. The method of construction of the third embodiment 100" differs slightly from the first embodiment 100, however. The third embodiment 100" includes a partially insulated conductor with an exposed tip that forms the first electrode 310 and the first insulative portion 330. The second electrode 320 is provided as a conductive sleeve 326, of a desired length 324, such as about 10 millimeters (mm), that may be crimped or otherwise fastened to the insulator of the probe 18. The second electrode 320 is electrically coupled to the electrical stimulation generation circuitry through an electrical filament or wire 328, which may be disposed within or coupled to the insulated portion of the sheath 18.

The first conductive surface 312 also preferably extends along a desirable length 315, longitudinally distally from the first insulative portion 330. A preferred range of lengths 215 is from 0.00 millimeters to about 6.00 millimeters, with about 0.50 to about 5.10 millimeters being most preferred. The length 315 may be selected on the basis of a desired expected charge density to be experienced during use. Preferred cathodic charge density may be in the range of about 0.5 µC/mm2 to about 2.0 µC/mm2, while preferred anodic charge density may be in the range of greater than zero µC/mm2 to about 0.6 µC/mm2. During use, it may be desirable to prevent tissue damage, in which case the size of the first electrode 310 and/or second electrode 320 should be sized accordingly. A preferred surface area of a cathodic surface, either the conductive surface 312 of the first electrode 310 or the conductive surface 322 of the second electrode 320, is generally about 1 mm² to about 20 mm², with about 1.6 mm² to about 16 mm² being most preferable.

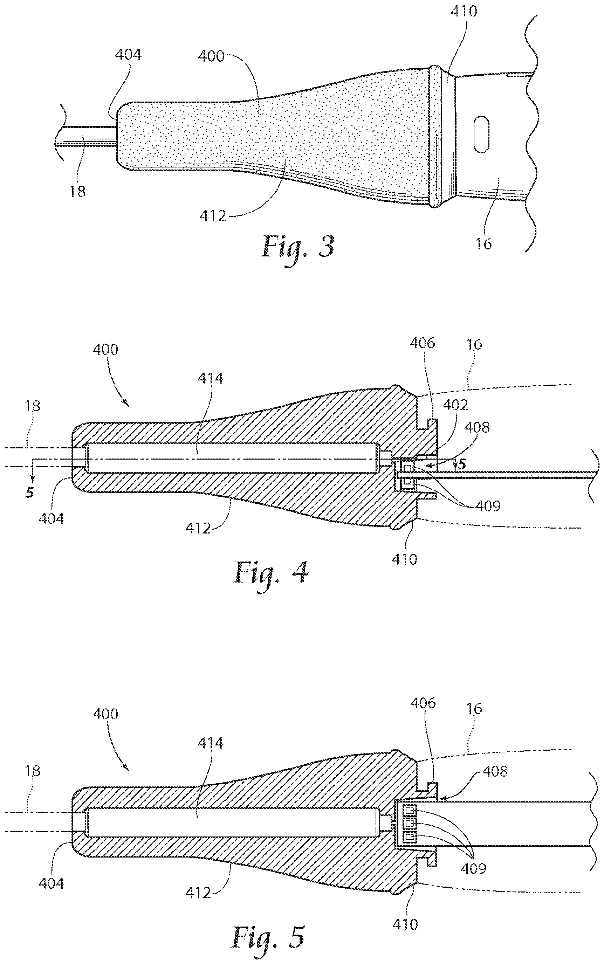

Turning now to Figures 3-5, an improved user interface may be described. The improved user interface comprises an improved status indicator interface including a shroud 400, which may be provided at and preferably surrounding or spanning the mechanical coupling of the probe 18 to the handle 16. The shroud 400 extends from a proximal mounting end 402 to a distal probe end 404. At the mounting end 402, the shroud 400 preferably includes a mounting flange or clip 406 to interface with and be captured by a portion of the handle 16. Alternatively, the shroud 400 may be formed integrally with the handle 16. Preferably formed longitudinally into the mounting end 402 is an indicator port 408 which may be adapted to at least partially surround one or more light sources 409, such as one or more light emitting diodes that are electrically coupled to the electrical stimulation generation circuitry in the handle 16. Disposed radially outwardly from the indicator port 408, and preferably on the external surface of the shroud 400, is a frustoconical lens surface 410, which may be polished. The frustoconical surface 410 helps to direct light from one or more light source 409 disposed at least partially within the indicator port 408 proximally towards the proximal end 12 of the device 10. The surface 410 preferably extends about an entire circumference of the shroud 400, though only a portion of the circumference may be used. The shroud 400 may also be provided without the frustoconical lens surface 410. The shroud 400 may be molded or otherwise formed, preferably as a unitary member, that is transparent or translucent. While the frustoconical surface 410 may be polished to enhance emission, the remainder of the external surface 412 of the shroud 400 is preferably textured in some way, such as by being bead-blasted, so as to assist in diffusion of light generated by the light sources 409. One or more of the indicated features assists in user visibility of light indications generated by the device. Formed into the shroud 400 is a probe bore 414 which allows passage of the probe 18 and/or electrical conductors (e.g. 116) into the shroud 400 to pass through to the circuitry in the handle 16.

The operation of one or more light sources 409 as a status indicator may be accomplished in a number of ways. For instance, visual indication provided by one or more light source 409 may allow an operator of the device 10 to confirm delivery or transmission of stimulus current. Through the use of different light colors, different flash rates, etc., the improved user interface may allow the operator to confirm that the instrument is powered on, and whether stimulus current is flowing. Thus the operator may have a greater confidence that, where there exists a failure to elicit a desired neurological response (e.g., a muscle contraction), such failure is more likely because of lack of viable nervous tissue near or contacted by the tip (e.g. 112) of the stimulator 50 rather than equipment malfunction, power failure, or other instrumentation problems.

As a representative example, one or more, or all light sources 409 may be configured to illuminate continuously in one color when the power is applied to the electrical stimulation circuitry within the handle 16 but the probe 18 is not in contact with tissue. After contact with tissue is made, one or more light sources 409 may flash (i.e., blink) or pulsate (i.e., change brightness intensity by dimming and/or brightening) to indicate that stimulation is available for delivery (i.e. stimulation would flow between the first electrode 110 and second electrode 120 if sufficient conductivity therebetween is established). If the stimulation has been requested, (i.e., the stimulation is available for delivery, but there is no stimulation being delivered because of a lack of sufficient conductivity between the first electrode 110 and the second electrode 120), one or more light sources 409 may illuminate in a different color, and/or may illuminate continuously or may flash.

By way of further example, one or more light sources 409 may operate in the following manner. One or more light sources 409 may provide a first indication that the stimulator 10 has not yet been turned on, that the stimulator power source has been depleted, that the stimulator has been used beyond some predetermined life span, or that an error has occurred thereby preventing stimulation. One or more light sources 409 may provide a second indication that the stimulator has been activated or powered on, but further that stimulation is not available for delivery. One or more light sources 409 may provide a third indication that the stimulator has been activated or powered on, and that greater than or equal to a first predetermined threshold of stimulus current is verified as being conducted between the first electrode and the second electrode. One or more light sources 409 may provide a third indication that the stimulator has been activated or powered on, and that not more than a second predetermined threshold of stimulus current is verified as being conducted between the first electrode and the second electrode.

The mentioned indications provided by one or more light sources 409 may include display styles such as the light sources being off (i.e. not emitting light), a single color at a single intensity, intermittency (blinking) of a single color at a substantially single intensity, a single color at a varying intensity (pulsing), intermittency of a single color at varying intensity (pulsed blinking), or similar operation using a plurality of colors. For instance, the first indication is preferably provided by keeping all light sources off (i.e. not emitting any light). The second indication is preferably provided by one or more, or all, light sources 409 emitting a yellow light at a single intensity. The third indication is preferably provided by one or more, or all, light sources 409 emitting a blinking yellow light at a substantially single intensity. The third indication is preferably provided by one or more, or all, light sources 409 emitting a blinking red light at a substantially single intensity.

The present invention is set out in the appended claims. The embodiments, aspects or examples according to the present description that do not fall within the scope of said claims are provided for illustrative purposes only and do not form part of the present invention.

## Claims

1. A handheld electrical stimulator (10) comprising:
a handle (16) extending from a proximal end (12) to a distal end;
a probe (18, 100, 100', 100") coupled to and extending away from the distal end of the handle;
electrical stimulation circuitry disposed substantially within the handle;
a first electrode (110, 210, 310) disposed at a distal end (14) of the probe and comprising a first conductive surface (112), wherein the first electrode is in electrical communication with the electrical stimulation circuitry;
a second electrode (120, 220, 320) disposed on the probe and comprising a second conductive surface (122), wherein the second electrode is electrically insulated from the first electrode and is in electrical communication with the electrical stimulation circuitry; and
a visual status indicator comprising a translucent shroud (400) having a proximal mounting end (402) coupled to the handle at a location proximal to the second electrode, an indicator port (408) formed longitudinally into the mounting end (402), and one or more light sources (409) disposed at least partially within the indicator port (408), wherein the shroud comprises a frustoconical lens surface (410) disposed about an entire perimeter of the shroud, and
wherein at least a portion of the probe that is proximal to the distal end of the handle passes through an aperture (414) of the visual status indicator.

2. The handheld stimulator according to claim 1, wherein the probe is straight along its entire length.

3. The handheld stimulator according to claim 1, wherein the shroud extends away from the distal end of the handle and towards the distal end of the probe.

4. The handheld stimulator according to claim 1, wherein the first and second electrodes are cylindrical in shape.

5. The handheld stimulator according to claim 1, wherein the first electrode is a solid cylinder.

6. The handheld stimulator according to claim 1, wherein the second electrode is cylindrical in shape and comprises an aperture formed therethrough.

7. The handheld stimulator according to claim 6, wherein the first electrode is a solid cylinder.

8. The handheld stimulator according to claim 7, wherein the probe is straight along its entire length and the first and second electrodes are disposed coaxially with each other.

9. The handheld stimulator according to claim 1, wherein the surface area of the second conductive surface (122) is greater than the surface area of the first conductive surface (112).

10. The handheld stimulator according to claim 9, wherein the surface area of the second conductive surface (122) is five times to nine times surface area of the first conductive surface (112).

11. The handheld stimulator according to claim 1, wherein the electrical stimulation circuitry is configured for generating an electrical charge at the first electrode at a charge density level of 0.5 to 2.0 micro-coulombs.

12. The handheld stimulator according to claim 11, wherein the light source is configured to generate an indication in response to a lack of sufficient conductivity between the first electrode and the second electrode.

13. The handheld stimulator according to claim 1, wherein an external surface of the shroud (400) is textured to diffuse light from the one or more light sources (409).

## Patentansprüche

1. Ein tragbarer bzw. handgeführter elektrischer Stimulator (10), der Folgendes umfasst:
einen Griff (16), der sich von einem proximalen Ende (12) zu einem distalen Ende erstreckt;
eine Sonde (18, 100, 100', 100"), die mit dem distalen Ende des Griffs gekoppelt ist und sich von diesem weg erstreckt;
eine elektrische Stimulationsschaltungsanordnung, die im Wesentlichen innerhalb des Griffs angeordnet ist;
eine erste Elektrode (110, 210, 310), die an einem distalen Ende (14) der Sonde angeordnet ist und eine erste leitende Oberfläche (112) umfasst, wobei die erste Elektrode in elektrischer Verbindung mit der elektrischen Stimulationsschaltungsanordnung steht;
eine zweite Elektrode (120, 220, 320), die auf der Sonde angeordnet ist und eine zweite leitende Oberfläche (122) umfasst, wobei die zweite Elektrode elektrisch von der ersten Elektrode isoliert ist und in elektrischer Verbindung mit der elektrischen Stimulationsschaltungsanordnung steht; und
eine optische Statusanzeige, die Folgendes umfasst: eine lichtdurchlässige Abdeckung (400) mit einem proximalen Befestigungsende (402), das mit dem Griff an einer Stelle proximal zur zweiten Elektrode gekoppelt ist, eine Anzeigeöffnung (408), die in Längsrichtung in das Befestigungsende (402) eingeformt ist, und eine oder mehrere Lichtquellen (409) umfasst, die zumindest teilweise innerhalb der Anzeigeöffnung (408) angeordnet sind,
wobei die Abdeckung eine kegelstumpfförmige Linsenoberfläche (410) umfasst, die um einen gesamten Umfang der Abdeckung angeordnet ist, und
wobei mindestens ein Abschnitt der Sonde, der proximal zum distalen Ende des Griffs liegt, eine Öffnung (414) der visuellen Statusanzeige durchsetzt.

2. Der tragbare Stimulator nach Anspruch 1, wobei die Sonde über ihre gesamte Länge gerade ist.

3. Der tragbare Stimulator nach Anspruch 1, wobei sich die Abdeckung vom distalen Ende des Griffs weg und zum distalen Ende der Sonde hin erstreckt.

4. Der tragbare Stimulator nach Anspruch 1, wobei die erste und zweite Elektrode zylindrisch geformt sind.

5. Der tragbare Stimulator nach Anspruch 1, wobei die erste Elektrode ein fester Zylinder ist.

6. Der tragbare Stimulator nach Anspruch 1, wobei die zweite Elektrode zylindrisch geformt ist und eine durch sie hindurch gebildete Öffnung umfasst.

7. Der tragbare Stimulator nach Anspruch 6, wobei die erste Elektrode ein fester Zylinder ist.

8. Der tragbare Stimulator nach Anspruch 7, wobei die Sonde über ihre gesamte Länge gerade ist und die erste und zweite Elektrode koaxial zueinander angeordnet sind.

9. Der tragbare Stimulator nach Anspruch 1, wobei der Flächeninhalt der zweiten leitenden Oberfläche (122) größer ist als der Flächeninhalt der ersten leitenden Oberfläche (112).

10. Der tragbare Stimulator nach Anspruch 9, wobei der Flächeninhalt der zweiten leitenden Oberfläche (122) fünfmal bis neunmal so groß ist wie der Flächeninhalt der ersten leitenden Oberfläche (112).

11. Der tragbare Stimulator nach Anspruch 1, wobei die elektrische Stimulationsschaltungsanordnung konfiguriert ist, um eine elektrische Ladung an der ersten Elektrode zu erzeugen, bei einer Ladungsdichte zwischen 0,5 und 2,0 Mikrocoulomb.

12. Der tragbare Stimulator nach Anspruch 11, wobei die Lichtquelle konfiguriert ist, um eine Anzeige zu erzeugen, als Reaktion auf einen Mangel an ausreichender Leitfähigkeit zwischen der ersten Elektrode und der zweiten Elektrode.

13. Der tragbare Stimulator nach Anspruch 1, wobei eine Außenfläche der Abdeckung (400) strukturiert ist, um Licht von einer oder mehreren Lichtquellen (409) zu streuen.

## Revendications

1. Un stimulateur électrique portatif (10) comprenant :
une poignée (16) s'étendant d'une extrémité proximale (12) à une extrémité distale ;
une sonde (18, 100, 100', 100") couplée à l'extrémité distale de la poignée et s'étendant à partir de celle-ci ;
une circuiterie de stimulation électrique disposée essentiellement à l'intérieur de la poignée ;
une première électrode (110, 210, 310) disposée à une extrémité distale (14) de la sonde et comprenant une première surface conductrice (112), sachant que la première électrode est en communication électrique avec la circuiterie de stimulation électrique ;
une deuxième électrode (120, 220, 320) disposée sur la sonde et comprenant une deuxième surface conductrice (122), sachant que la deuxième électrode est électriquement isolée de la première électrode et qu'elle est en communication électrique avec la circuiterie de stimulation électrique ; et
un indicateur d'état visuel comprenant une enveloppe translucide (400) ayant une extrémité de montage proximale (402) couplée à la poignée à un emplacement proche de la deuxième électrode, un orifice d'indicateur (*indicator port*) (408) formé longitudinalement dans l'extrémité de montage (402), et une ou plusieurs sources de lumière (409) disposées au moins partiellement dans l'orifice d'indicateur (408),
sachant que l'enveloppe comprend une surface optique tronconique (410) disposée sur un périmètre entier de l'enveloppe, et
sachant qu'au moins une partie de la sonde, qui est proximale à l'extrémité distale de la poignée, passe à travers une ouverture (414) de l'indicateur d'état visuel.

2. Le stimulateur portatif d'après la revendication 1, sachant que la sonde est droite sur toute sa longueur.

3. Le stimulateur portatif d'après la revendication 1, sachant que l'enveloppe s'étend depuis l'extrémité distale de la poignée vers l'extrémité distale de la sonde.

4. Le stimulateur portatif d'après la revendication 1, sachant que les électrodes première et deuxième sont de forme cylindrique.

5. Le stimulateur portatif d'après la revendication 1, sachant que la première électrode est un cylindre solide.

6. Le stimulateur portatif d'après la revendication 1, sachant que la deuxième électrode est de forme cylindrique et comprend une ouverture formée à travers celle-ci.

7. Le stimulateur portatif d'après la revendication 6, sachant que la première électrode est un cylindre solide.

8. Le stimulateur portatif d'après la revendication 7, sachant que la sonde est droite sur toute sa longueur et que les électrodes première et deuxième sont disposées coaxialement l'une par rapport à l'autre.

9. Le stimulateur portatif d'après la revendication 1, sachant que la superficie de la deuxième surface conductrice (122) est supérieure à la superficie de la première surface conductrice (112).

10. Le stimulateur portatif d'après la revendication 9, sachant que la superficie de la deuxième surface conductrice (122) est cinq fois à neuf fois plus grande que la superficie de la première surface conductrice (112).

11. Le stimulateur portatif d'après la revendication 1, sachant que la circuiterie de stimulation électrique est configurée pour générer une charge électrique à la première électrode à un niveau de densité de charge de 0,5 à 2,0 micro-coulombs.

12. Le stimulateur portatif d'après la revendication 11, sachant que la source lumineuse est configurée pour générer une indication en réponse à un manque de conductivité suffisante entre la première électrode et la deuxième électrode.

13. Le stimulateur portatif d'après la revendication 1, sachant qu'une surface externe de l'enveloppe (400) est texturée pour diffuser la lumière d'une ou de plusieurs sources lumineuses (409).
